(19) **Europäisches Patentamt** / **European Patent Office** / **Office européen des brevets**

(11) **EP 4 392 405 B1**

(12) **EUROPEAN PATENT SPECIFICATION**

(45) Date of publication and mention of the grant of the patent:
**28.01.2026 Bulletin 2026/05**

(51) International Patent Classification (IPC):
**C07C 273/14** (2006.01)   **B01D 53/56** (2006.01)
**B01D 53/86** (2006.01)   **C01C 1/02** (2006.01)
**C01C 1/26** (2006.01)

(21) Application number: **22769240.7**

(22) Date of filing: **26.08.2022**

(52) Cooperative Patent Classification (CPC):
**C07C 273/14; C01C 1/02; C01C 1/26;** B01D 53/56;
B01D 53/8625; B01D 2251/206

(86) International application number:
**PCT/EP2022/073745**

(87) International publication number:
**WO 2023/025922 (02.03.2023 Gazette 2023/09)**

(54) **METHOD FOR PRODUCING A SNCR/SCR SOLUTION**

VERFAHREN ZUR HERSTELLUNG EINER SNCR-/SCR-LÖSUNG

PROCÉDÉ DE PRODUCTION D'UNE SOLUTION SNCR/SCR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.08.2021 EP 21193197**

(43) Date of publication of application:
**03.07.2024 Bulletin 2024/27**

(73) Proprietor: **Yara International ASA**
**0277 Oslo (NO)**

(72) Inventors:
• **PORRO, Lino Giovanni**
**1040 Etterbeek (BE)**
• **VAN BELZEN, Ruud**
**4541 HJ Sluiskil, Terneuzen (NL)**

• **MALO, Patrice**
**76700 Gonfreville-l'Orcher (FR)**
• **KOHNKE, Sven**
**25572 Buettel (DE)**
• **RUNGGER, Werner**
**39030 San Martino in Badia (BZ) (IT)**
• **WEIGL, Klaus**
**2533 Klausen-Leopoldsdorf (AT)**
• **DUPONCHEL, Vincent**
**1210 Brussel (BE)**

(74) Representative: **De Clercq & Partners**
**Edgard Gevaertdreef 10a**
**9830 Sint-Martens-Latem (BE)**

(56) References cited:
**WO-A1-98/42623   US-B2- 10 633 331**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of the invention

The present application is related to a method for producing an aqueous composition, wherein the aqueous composition is to be used in a selective catalytic or non-catalytic reduction process.

### Background of the invention

A selective non-catalytic reduction process, or SNCR, is a process wherein a nitrogen-containing compound, such as ammonia ($NH_3$) or urea, is used as an aqueous composition or in solid form to reduce the content in nitrogen oxides, NOx, of a flue gas. The nitrogen-containing compound is added to the stream of flue gas and heated up to a temperature of from 700°C to 1100 °C. Ammonia reacts with the NOx in the presence of oxygen via a redox reaction and nitrogen gas and water are obtained.

A selective catalytic reduction process, or SCR, is a process wherein a nitrogen-containing compound, such as ammonia or urea, is used as an aqueous composition or in solid form to reduce the content in nitrogen oxides, NOx, of a flue gas in the presence of a catalyst. The reaction temperature can be reduced significantly compared to a SNCR process: the typical operation window is between 180°C and 450°C. As in the SNCR process, ammonia reacts with the NOx in the presence of oxygen via a redox reaction and nitrogen gas and water are obtained.

When urea is used in a SNCR or SCR process, it is first decomposed to ammonia and carbon dioxide, and the ammonia obtained reacts as described above.

Flue gas from industrial plants burning organic material, such as waste incinerators, or waste-to-energy plants, or cement plants, can be treated by these methods and reduces significantly the emissions of greenhouse gases of these plants.

Aqueous solutions of ammonia or urea are the most common nitrogen sources used in SNCR and SCR systems.

However, there is always a need to produce new compositions that are suitable to be used as a ammonia source in SNCR/SCR processes, in particular using waste streams or secondary streams from industrial plants.

### Summary of the invention

The present disclosure provides a cheap and simple method to prepare an aqueous solution comprising ammonia as ammonium salts, wherein the aqueous solution is suitable to be used in SNCR and SCR systems.

As used herein, a "SNCR/SCR solution" refers to a solution suitable to be used in a SNCR or SCR system. It has been discovered that it was possible to prepare a SNCR/SCR solution using waste streams produced in an industrial plant that uses ammonia and carbon dioxide as raw material, in particular in a plant producing urea.

In a first aspect, the present disclosure provides a method for producing a selective non-catalytic reduction /selective catalytic reduction (SNCR/SCR) solution, comprising the steps of: a) reacting ammonia and carbon dioxide in a reactor, thereby obtaining an aqueous composition comprising urea and ammonium salts; b) processing the aqueous composition obtained in step a), thereby obtaining a first aqueous solution comprising urea, and a second aqueous solution comprising ammonium salts; and c) diluting the second aqueous solution comprising ammonium salts obtained in step b) with a third aqueous solution, thereby producing the SNCR/SCR solution.

In another aspect, the present disclosure provides the use of an aqueous stream comprising ammonium salts from a urea-producing plant for producing a SNCR/SCR solution.

### Brief description of the figures

The following description of the figure of a specific embodiment of a system according to the present disclosure is only given by way of example and is not intended to limit the present explanation, its application or use. In the figure, identical reference numerals refer to the same or similar parts and features.

Figure 1 describes an embodiment of the method according to the present disclosure in a urea plant based on the Stamicarbon technology.
Figure 2 describes an embodiment of the method according to the present disclosure in a urea plant based on the Saipem technology.
Figure 3 describes an embodiment of the method according to the present disclosure in a urea plant based on the Montedison IDR technology.

## Detailed description of the invention

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

All references cited in this description are hereby deemed to be incorporated in their entirety by way of reference.

As used herein, the following terms have the following meanings:

"A", "an", and "the" as used herein refers to both singular and plural referents unless the context clearly dictates otherwise. By way of example, "a component" refers to one or more than one component.

"About" as used herein referring to a measurable value such as a parameter, an amount, a temporal duration, and the like, is meant to encompass variations of +/-20 % or less, in particular +/-10 % or less, more in particular +/-5 % or less, even more in particular +/-1 % or less, and still more in particular +/-0.1 % or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

The expression "weight percent", "%wt" or "weight%", here and throughout the description unless otherwise defined, refers to the relative weight of the respective component based on the overall weight of the formulation.

In a first aspect, the present disclosure provides a method for producing a selective non-catalytic reduction /selective catalytic reduction (SNCR/SCR) solution, comprising the steps of: a) reacting ammonia and carbon dioxide in a reactor, thereby obtaining an aqueous composition comprising urea and ammonium salts; b) processing the aqueous composition obtained in step a), thereby obtaining a first aqueous solution comprising urea, and a second aqueous solution comprising ammonium salts; and c) diluting the second aqueous solution comprising ammonium salts obtained in step b) with a third aqueous solution, thereby producing the SNCR/SCR solution. Urea is a small organic molecule obtained by the reaction between two molecules of ammonia and one molecule of carbon dioxide (see Equation 1). The reaction also produces water:

$$2\ NH_3 + CO_2 \rightarrow NH_2CONH_2 + H_2O \qquad\qquad (\text{Equation 1})$$

Today, urea is produced in large industrial plants wherein the first step is to mix ammonia and carbon dioxide under high pressure (for example, from 130 to 240 bar) and high temperatures (for example, from 170 to 190 °C) in a reactor, in particular a reactor in a high pressure section of a urea-producing plant. The solution produced by the high pressure section is an aqueous solution comprising urea, and ammonium salts. The reaction forming urea (see Equation 1) is an equilibrium, and it is not possible to obtain a full conversion of the starting materials into urea.

When ammonia and carbon dioxide are dissolved in an aqueous solution, they can form three different ammonium salts depending on several factors, such as the temperature of the solution, and the content in ammonia and in carbon dioxide. The three possible salts are ammonium bicarbonate ($NH_4HCO_3$), ammonium carbonate (($NH_4)_2CO_3$), and ammonium carbamate ($NH_2CO_3NH_4$). Moreover some free ammonia can be present in the aqueous solution. As used herein, a solution comprising ammonium salts may comprise one or more compounds selected from the group consisting of ammonium bicarbonate, ammonium carbonate, and ammonium carbamate.

The solution produced from the reactor, in particular a reactor from the high pressure section of a urea-producing plant, has then to be processed to remove the ammonium salts contained therein, and concentrated to obtain a urea solution. The clean, concentrated urea solution may then be used to prepare solid urea-based products, such as urea prills or granules, or liquid urea-based aqueous solutions, such as Diesel Exhaust Fluid (DEF), or other products, such as urea formaldehyde. During these processing steps, the ammonia and carbon dioxide comprised in the solution as ammonium salts, and optionally as free ammonia, are recovered in waste streams. These waste streams may be further processed, and are very often re-directed to the high pressure section via a return loop, so that they react again and produce more urea. This return loop increases the overall conversion rate of the raw materials into urea.

Today, there are different models of urea plants depending on the manufacturer of the plant. For example, companies such as Stamicarbon, Saipem, and Casale, each provide different technologies for urea production. In addition, several existing urea plants use the Montedison IDR technology. However, all urea plants operate on the principle of first reacting ammonia and carbon dioxide to obtain a mother solution containing urea and by-products and then purifying and concentrating this mother solution. The different plant models vary on how this mother solution is processed to obtain a concentrated urea solution.

Once the by-products, i.e. ammonium salts, are removed from the mother solution containing urea, they are re-injected in the high pressure section as an aqueous solution, and water is detrimental to the urea-forming reaction. Water is a byproduct of the urea-forming reaction (Equation 1), so adding water to the high pressure section pushes the equilibrium of the reaction towards the starting materials, which is not desirable. Simply discarding the by-products is not economically interesting.

It was now found out that a part or the totality of the streams comprising ammonium salts produced by a urea-producing plant could be used to produce a SNCR/SCR solution.

Instead of being re-injected in the high pressure section, the aqueous solutions comprising ammonium salts produced in the production process could be transformed into SNCR/SCR solutions. This reduces the introduction of additional water in the high pressure section and increases the conversion rate of the urea synthesis step.

In most urea-producing plants, aqueous solutions comprising ammonium salts and optionally urea are also produced by other equipment than the synthesis section. For example, an aqueous solution comprising ammonium salts may be obtained from a medium-pressure or low-pressure section of a plant producing urea, in particular from a condenser comprised in a medium-pressure or low-pressure section of a plant producing urea. Such aqueous solution is usually re-introduced in the synthesis section or a medium-pressure section, but can now be transformed into a SNCR/SCR solution instead.

An aqueous solution comprising ammonium salts may also be obtained from the hydrolyser section of a plant producing urea.

In one embodiment, the second aqueous solution comprising ammonium salts obtained in step b) comprises from 1 to 50 weight%, from 10 to 50 weight%, from 2 to 50 weight%, from 1 to 40 weight%, from 10 to 40 weight%, from 20 to 40 weight%, from 1 to 35 weight%, from 10 to 35 weight%, from 20 to 35 weight%, from 25 to 40 weight%, or from 25 to 35 weight% of nitrogen, expressed as ammonia.

In one embodiment, the second aqueous solution comprising ammonium salts obtained in step b) comprises from 1 to 50 weight%, from 10 to 50 weight%, from 2 to 50 weight%, from 1 to 40 weight%, from 10 to 40 weight%, from 20 to 40 weight%, from 1 to 35 weight%, from 10 to 35 weight%, from 20 to 35 weight%, from 25 to 40 weight%, from 25 to 35 weight%, of nitrogen, from 1 to 10 weight%, from 1 to 20 weight%, from 1 to 30 weight%, from 5 to 40 weight%, from 5 to 30 weight%, from 5 to 20 weight%, from 5 to 15 weight% of carbon, expressed as carbon dioxide.

The second aqueous solution comprising ammonium salts obtained in step b) is diluted with a third aqueous solution to adjust the properties of the solution, in particular the weight content, or concentration, of nitrogen and carbon of the solution.

The aqueous composition can then be used directly in a SNCR/SCR system or stored over an extended period before being transported and used.

In one embodiment, the method is performed in a plant producing urea.

In one embodiment, the aqueous composition obtained in step c) comprises from 10 to 40 weight%, from 10 to 35 weight%, from 10 to 30 weight%, from 12 to 40 weight%, from 12 to 35 weight%, from 12 to 30 weight%, from 15 to 40 weight%, from 15 to 35 weight%, or from 15 to 30 weight% of nitrogen, expressed as ammonia. It was found that a nitrogen content of from 10 to 40 weight%, expressed as ammonia, was a suitable SNCR/SCR solution. Furthermore, this content can be obtained from a large majority of streams produced in a plant producing urea.

In one embodiment, the aqueous composition obtained in step c) comprises from 5.0 to 40 weight%, from 5.0 to 35 weight%, from 5.0 to 30 weight%, from 5.0 to 25 weight%, from 7.0 to 35 weight%, from 7.0 to 30 weight%, from 7.0 to 25 weight%, from 8.0 to 35 weight%, from 8.0 to 30 weight%, from 8.0 to 25 weight%, of carbon, expressed as carbon dioxide.

In one embodiment, wherein the aqueous composition obtained in step c) does not form crystals above 15°C. It may be preferable that the aqueous composition obtained in step c) does not form crystals above 15°C, as crystals disturb the flowability of the solution. Additives may be added to the aqueous composition to lower the temperature at which crystals appear in the aqueous composition.

In one embodiment, the crystallization temperature of the aqueous composition obtained in step c) is from 0 to 15 °C, in particular from 0 to 10 °C. As used herein, the crystallization temperature of a solution refers to the temperature where crystals start to appear in the solution that is slowly cooled down from ambient temperature.

In one embodiment, the aqueous composition obtained in step c) has a boiling point of at least 35 °C, at least 40 °C, at least 45 °C, or at least 50 °C. Solutions comprising ammonia as free ammonia or ammonium salts may have a boiling point below 100 °C. For safety reasons, it may be preferable that the aqueous composition obtained in step c) has a boiling point of at least 35 °C, i.e. of 35 °C or above, so that the aqueous composition does not boil at ambient temperature.

In one embodiment, the vapor pressure at ambient temperature or at a temperature of at most 35 °C, of the aqueous composition obtained in step c) is below 1 atm or 1.01 bar. A low vapor pressure ensures that the aqueous composition does not boil under storage and/or transport and increases the safety profile of the aqueous composition.

In one embodiment, step b) comprises generating a gaseous stream comprising water, ammonia, and carbon dioxide from the aqueous composition comprising urea and ammonium salts, and condensing the gaseous stream comprising water, ammonia, and carbon dioxide into the second aqueous solution comprising ammonium salts.

In one embodiment, the step of removing ammonium salts from an aqueous solution comprising urea comprises generating a gaseous stream comprising water, ammonia, and carbon dioxide from the aqueous composition comprising urea and ammonium salts. Since ammonia and carbon dioxide are gases within certain conditions of temperature and pressure, for example above -78 °C at atmospheric pressure, it is possible to transform the ammonium salts into their neutral molecule, which then can be removed from the aqueous solution. Once the ammonia and carbon dioxide are in gaseous phase, a second step may be performed wherein the gases are re-condensed in an aqueous solution. By doing these two steps, an amount of the ammonium salts comprised in the aqueous solution comprising urea and ammonium salts have been removed from the urea solution and recovered in another aqueous solution.

In one embodiment, generating a first aqueous solution comprising urea and a gaseous stream comprising water, ammonia, and carbon dioxide from the aqueous composition comprising urea and ammonium salts, is achieved by heating up the aqueous composition comprising urea and ammonium salts obtained in step a). It is known that an aqueous solution comprising urea and ammonium salts, may be heated up, for example with steam, so that the ammonium salts dissociate into ammonia and carbon dioxide. Within the same step or with a separate equipment, the dissolved free ammonia and carbon dioxide can be removed from the aqueous solution. This heating step may be performed in an apparatus called a stripper.

In one embodiment, the amount of nitrogen comprised in the SNCR/SCR solution obtained in step c) represents from 0.5% to 10%, from 0.5% to 8%, from 0.% to 6%, or from 0.5% to 4.0% of the amount of nitrogen introduced, as ammonia, in a urea-producing plant. The amount of nitrogen comprised in the aqueous composition obtained in step c) may represent an amount of the total nitrogen introduced in the plant that varies over time, as this may depend on factors external to the plant, such as demand in urea-based products and demand in solution for SNCR/SCR.

In one embodiment, the molar ratio of ammonia to carbon dioxide of the aqueous composition obtained in step c) is from 2 to 10. The molar ratio of the SNCR/SCR solution may depend on the composition of the second aqueous composition obtained in step b) and on the composition of the third aqueous composition used in step c).

In one embodiment, the aqueous composition obtained in step c) comprises urea, in particular from 0.01 to 5.0 weight%, from 0.01 to 2.0 weight% or from 0.01 to 1.0 weight% of urea. The aqueous composition for SNCR/SCR obtained in step c) may comprise urea. Some equipment used to remove ammonium salts from aqueous solutions comprising urea and ammonium salts may not be completely selective and may remove some urea along with ammonia and carbon dioxide. Urea may also be formed during the separation of ammonia and carbon dioxide from aqueous solutions.

In one embodiment, the aqueous composition obtained in step c) comprises methanol and/or formaldehyde, in particular from 0.01 to 5.0 weight%, from 0.01 to 2.0 weight% or from 0.01 to 1.0 weight% of methanol. The aqueous composition for SNCR/SCR may comprise other chemicals used in the plant, that may enter the aqueous streams comprising ammonium salts, and/or that may be present in the third aqueous solution used to dilute the aqueous solution comprising ammonium salts.

In one embodiment, the third aqueous solution used in step c) is selected from the group consisting of demineralized water, steam condensate, process condensate, and any mixture thereof. The second aqueous solution comprising ammonium salts obtained in step b) has a concentration in ammonium salts higher than the recommended amount for SNCR/SCR use, and the aqueous solution should be diluted before being used in an SNCR/SCR system. Different water sources available in a plant producing urea may be used. Demineralized water has the benefit that it is clean and does not contain any impurities, however, it may also be more expensive than other water sources. Plants producing urea have an extensive steam networks with multiple steam-producing units and multiple steam-consuming units. Steam consumed in some units is condensed as steam condensate and cooled down before being sent out of the plant. It may be an advantage to use steam condensate produced in the plant, to obtain an aqueous solution essentially consisting of water. This reduces the operating cost of the plant because plants have to pay to dispose of waste streams. The steam condensate can then be added as such or further mixed with water or another aqueous solution to the second aqueous solution comprising ammonium salts obtained in step b) to obtain an SNCR/SCR solution.

Process condensate refers to the water produced by the urea-forming reaction (see Equation 1) that is gradually removed from the urea solution. Process condensate can be clean condensate, without any $NH_3$, $CO_2$ and urea or untreated condensate comprising up to 10 weight% $NH_3$, and/or up to 5 weight% $CO_2$ and/or up to 2 weight% urea. Process condensate may comprise from 0 to 10 weight% of nitrogen, expressed as $NH_3$. Process condensate may comprise from 0 to 5.0 weight% of carbon, expressed as $CO_2$. Process condensate may comprise from 0 to 2.0 weight% of urea.

In one embodiment, the third aqueous solution used in step c) may comprise ammonium salts, such as of ammonium bicarbonate, ammonium carbonate, and/or ammonium carbamate and optionally free ammonia. The third aqueous solution used to dilute the aqueous solution may comprise ammonium salts suitable to be used in a SNCR/SCR process.

In another aspect, the present disclosure provides the use of an aqueous stream comprising ammonium salts from a urea-producing plant for producing a SNCR/SCR solution.

In another aspect, the present disclosure provides a urea-producing plant comprising a high pressure section, a low pressure section, a return line for transporting a first aqueous solution comprising ammonium salts from the low pressure section to the high pressure section, a means for drawing out an amount of the first aqueous solution from the return line, and a means for mixing the first aqueous solution with a second aqueous solution.

The urea-producing plant is suitable for performing the method according to the present disclosure. All urea-producing plants comprise a high pressure section wherein urea and ammonia are combined to provide an aqueous solution comprising urea and by-products, such as ammonium salts. The plants also comprise a low-pressure section wherein the aqueous solution produced by the high pressure section is purified and concentrated. The plants also comprise a return line from the low pressure section to the high pressure section to re-inject the ammonia and carbon dioxide recovered in the low pressure section back into the high pressure section to increase the overall yield of the plant. This return line is transporting an aqueous solution comprising ammonium salts. The precise content in ammonia and carbon dioxide of the solution transported by the return line may vary from plant to plant, and/or may depend on the exact operating conditions of a particular plant. But this return line always transports an aqueous solution comprising ammonia and carbon dioxide in the form of ammonium salts, and optionally free ammonia. So, an existing plant can be modified by adding a means for drawing out an amount of the first aqueous solution from the return line, and a means for mixing the first aqueous solution with a second aqueous solution. The means for drawing out an amount of the first aqueous solution from the return line can be any means suitable for taking an amount of the aqueous solution transported in the return line and directing that amount to a different pipe. The means for mixing the first aqueous solution drawn out of the return line with a second aqueous solution can comprise a mixing tank where the first and second aqueous solutions are directed and mixed therein. The means for mixing may also be an injection means into the pipe carrying the first aqueous solution. The solution obtained after the means for mixing is a SNCR/SCR solution.

In another aspect, the present disclosure provides a urea-producing plant comprising a high pressure section, a low pressure section, a finishing section, a return line for transporting a first aqueous solution comprising ammonium salts from the finishing section to the low pressure section, a means for drawing out an amount of the first aqueous solution from the return line, and a means for mixing the first aqueous solution with a second aqueous solution.

Instead of drawing out a first aqueous solution from the return line between the high pressure section and the low pressure section, it may be possible to draw a first aqueous solution from the finishing section of a urea-producing plant and the low pressure section.

The finishing section and the return line to the low pressure section of a urea-producing plant may vary depending on the type of products produced in the plant and the technology used therein. For example, the return line from the finishing section to the low pressure section may comprise a desorption section or a hydrolyzer section. A return line always transports ammonia and carbon dioxide, but it may be under different states depending on the plant. The return line may either transport an aqueous composition comprising ammonium salts, and optionally free urea, or a gaseous stream comprising ammonia, carbon dioxide and water. In the case where the return line transports a gaseous composition, it may be required to install a device, such as a condenser, to condense the gaseous composition into an aqueous composition comprising ammonium salts.

In one embodiment, the urea-producing plant comprises a medium pressure section, a return line for transporting a third aqueous solution comprising ammonium salts from the low pressure section to the medium pressure section, a means for drawing out an amount of the third aqueous solution from the return line, and a means for mixing the third aqueous solution with a fourth aqueous solution. Some urea-producing plants also comprise a medium pressure section, and a return line from the low pressure section to the medium pressure section. It is possible to prepare an SNCR/SCR solution from the solution transported in this return line. By adding a means for drawing out an amount of the third aqueous solution from the return line, and a means for mixing the third aqueous solution with a fourth aqueous solution, it is now possible to produce a SNCR/SCR solution from a different source of the urea-producing plant.

In one embodiment, the finishing section is a solidification section, for example a prilling section or granulating section. The finishing section often comprises a device that produces an aqueous solution comprising ammonium salts, such as a scrubber. The scrubber solution is directed to the low-pressure section or the high-pressure section to be recycled. It is possible to use that solution produced by the finishing section to produce a SNCR/SCR solution.

In another aspect, the present disclosure provides a method for adapting an existing urea-producing plant, comprising the steps of installing a means for drawing out an amount of a first aqueous solution comprising ammonium salts, and installing a means for mixing the first aqueous solution with a second aqueous solution.

It is possible to adapt a conventional urea-producing plant into a plant capable of producing a SNCR/SCR solution. It is required to install two pieces of equipment: a means for drawing out an amount of a first aqueous solution comprising ammonium salts, and a means for mixing the first aqueous solution with a second aqueous solution. Firstly, the means for drawing out a first aqueous solution can be installed in different locations where a solution comprising ammonium salts is transported. As explained above, a urea-producing plant comprises several suitable locations, such as the return line between the low pressure section and the high pressure section, or between the low pressure section and the medium pressure section, if present, or between the medium pressure section, if present, and the high pressure section. It may also be possible to install the means for drawing out an aqueous solution in the return line between the finishing section and the low pressure section, or after any condenser that transforms a gaseous stream comprising ammonia, carbon dioxide and water into an aqueous solution comprising ammonium salts.

The present invention will now be described in different models of urea-producing plants, in particular plants operated by Yara, a fertilizer producer. The compositions of the various streams are given as examples, but may vary from one plant to another and depending on the production conditions.

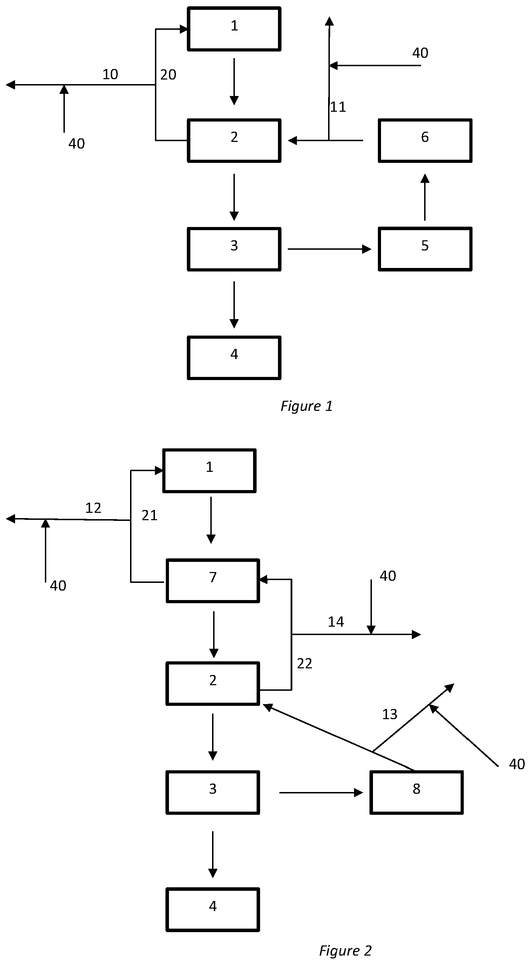

Figure 1 is a schematic representation of a urea-producing plant based on the Stamicarbon technology, such as the one located in Le Havre, France, and operated by Yara. This urea plant comprises a synthesis section **1,** a low-pressure section **2,** an evaporation section **3,** a finishing section **4,** a desorption section **5.** In the synthesis section **1,** comprising several equipment, carbon dioxide and ammonia gases are sent to the reactor operating at about 180 - 186 °C and 140 - 150 bar. The solution obtained from the synthesis section **1** comprises from 50 to 58 weight% of urea, 8 -12 weight% of nitrogen as ammonium salts and free ammonia, expressed as ammonia, and 8 - 12 weight% of carbon, expressed as carbon dioxide. The low-pressure section **2** comprises several equipment, and removes the ammonia and carbon dioxide from the solution to provide a urea solution depleted of ammonium salts and free ammonia to the evaporation section **3.** The low-pressure section **2** also generates an aqueous solution comprising ammonium salts, that is re-injected in the synthesis section, in particular the HP Scrubber, via a return loop **20** between the low-pressure section **2** and the synthesis section **1.** The evaporation section **3** reduces the water content of the urea solution to provide a urea melt to the finishing section **4,** which transforms the melt into solid urea particles in a prilling unit. Alternatively, a granulation unit could be used. The evaporation section **3** also produces a gaseous stream comprising ammonia and carbon dioxide that is directed to the desorption section **5,** which generates a further gaseous stream comprising ammonia and carbon dioxide. This gaseous stream is directed to the LP section **2** where it is condensed and sent to the synthesis section **1.** Alternatively, a low-pressure condenser **6** can be installed after the desorption section **5** . The low-pressure condenser **6** could generate an aqueous solution comprising ammonium salts from the gaseous stream produced by the desorption section **5.** The low-pressure condenser **6** may be connected to the low-pressure section **2.** An aqueous solution for SNCR/SCR can be obtained in two locations **10** and **11:** in the return loop **20** between the low-pressure section **2** and the synthesis section **1,** wherein the aqueous solution comprises from 30 to 40 weight% of nitrogen, expressed as ammonia, and from 35 to 40 weight% of carbon, expressed as $CO_2$, and the balance to 100 weight% of water, and in the connection between the low-pressure condenser **6** and the low-pressure section **2,** wherein the aqueous solution comprises from 30 to 35 weight% of nitrogen, expressed as ammonia, and from 5 to 15 weight% of carbon, expressed as $CO_2$, and the balance to 100 weight% of water. In these two connections, an aqueous composition comprising ammonium salts is transported, and a portion or the totality of that composition can be withdrawn and transformed into an SNCR/SCR solution after dilution with an aqueous solution **40.** The nitrogen and carbon content of these compositions vary depending on the exact running conditions of the plant. A chemical analysis can be performed to determine the nitrogen and carbon content of the composition and add a further aqueous solution to obtain the desired composition. For example, if the solution obtained from pipe **10** comprises 36 weight% of nitrogen, expressed as ammonia, 36 weight% of carbon, expressed as carbon dioxide, and 33 weight% of water, it can be diluted with demineralized water in a 2:1 ratio to give a solution comprising 24 weight% of nitrogen, expressed as ammonia, 24 weight% of carbon, expressed as carbon dioxide, and 52 weight% of water, which is suitable to be used as a SNCR/SCR solution.

Figure 2 is a schematic representation of a urea-producing plant based on the technology from Saipem, such as the one located in Barbrala, India, and another one located in Brunsbüttel, Germany, both operated by Yara. The plant comprises a synthesis section **1,** a medium-pressure (MP) section **7,** a low-pressure (LP) section **2,** an evaporation section **3,** a finishing section **4,** and a hydrolyser section **8.** Ammonia and carbon dioxide are mixed in the synthesis section **1,** comprising several equipment, and produce an aqueous solution comprising urea, ammonium salts and free ammonia, which is directed to the MP section **7.** The MP section produces an aqueous solution with a higher concentration in urea, and still comprising ammonium salts and free ammonia, which is sent to the LP section **2** and an aqueous stream comprising ammonium salts and free ammonia, in particular about 47 weight% of nitrogen, expressed as ammonia, 24 weight% of carbon, expressed as carbon dioxide, and less than 1 weight% of urea, which is produced by the MP condenser and is re-injected in the synthesis section **1** via the return loop **21.** The LP section **2** generates a concentrated urea solution depleted of ammonium salts, which is sent to the evaporation section **3,** and an aqueous composition comprising ammonium salts and free ammonia, in particular about 46 weight% of nitrogen, expressed as ammonia, 21 weight% of carbon, expressed as carbon dioxide, and less than 1 weight% of urea, which is produced by a LP condenser, and is re-injected in the MP section **7** via the return loop **22.** The evaporation section **3** produces a urea melt with a low water content, for example 1 to 5 weight%, which is sent to the finishing section **4** to produce urea prills, and a gaseous stream comprising mainly water and some ammonia, carbon dioxide and urea carry over, which is condensed and directed to the hydrolyzer section **8.** The hydrolyzer section **8** treats the condensates to yield a clean process condensate and an aqueous composition comprising ammonium salts and free ammonia, in particular about 44 weight% of nitrogen, expressed as ammonia, 10 weight% of carbon, expressed as carbon dioxide, and less than 1 weight% of urea, that may be re-injected in the low-pressure section **2.** An aqueous solution for SNCR/SCR can be obtained in three locations **12, 13** and **14:** in the return loop between the medium-pressure section **7** and the synthesis section **1,** between the low-pressure section **2** and the medium-pressure section **7** and between the hydrolyzer section **8** and the low-pressure section **2.** In these three connections, an aqueous composition comprising ammonium salts is transported, and a portion of that composition can be withdrawn and transformed into a SNCR/SCR solution after dilution with aqueous solution **40.** The nitrogen and carbon content of these compositions vary depending on the exact running conditions of the plant. A chemical analysis can be performed to determine the nitrogen and carbon content of the composition and add a further aqueous solution to obtain the desired composition. For example, the aqueous solution produced by the medium-pressure section **7** comprises about 47 weight% of nitrogen, expressed as ammonia, 24 weight% of carbon, and can be diluted with water in a 1:1 ratio to provide an aqueous solution about 23.5 weight% of nitrogen, expressed as ammonia, and 12 weight% of carbon, that is suitable to be used as a SNCR/SCR solution.

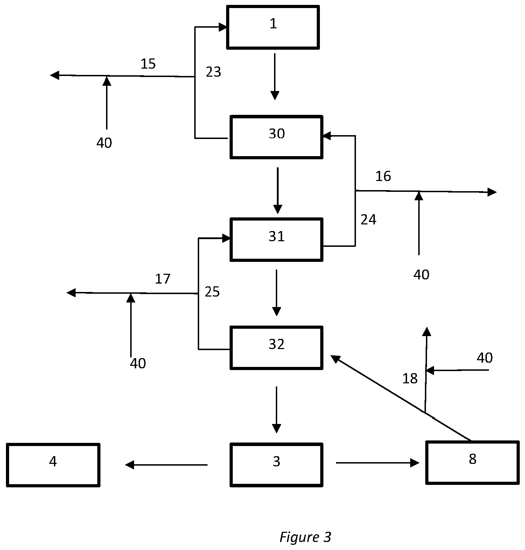

Figure 3 is a schematic representation of a urea-producing plant based on the technology from Montedison IDR, such as the one located in Ferrara, Italy, and operated by Yara. The plant comprises a synthesis section **1,** consisting of several equipment, a first-cycle section **30,** a second-cycle section **31,** a third-cycle section **32,** an evaporation section **3,** a finishing section **4** and a hydrolyzer section **8.** Ammonia and carbon dioxide are mixed in the synthesis section **1** and produce an aqueous solution comprising urea, ammonium salts and some free ammonia, which is directed to the first-cycle section **30.** The first-cycle section **30** produces an aqueous solution with a higher concentration in urea than the aqueous solution produced by the synthesis section **1,** and still comprising ammonium salts and some free ammonia, which is sent to the second-cycle section **31** and an aqueous stream comprising ammonium salts and free ammonia, in particular about 38 weight% of nitrogen, expressed as ammonia, 39 weight% of carbon, expressed as carbon dioxide, and less than 1 weight% of urea, which is produced by a condenser and is re-injected in the synthesis section **1** via the return loop **23.** The second-cycle section **30** produces an aqueous solution with a higher concentration in urea than the aqueous solution produced by the first-cycle section **30,** and still comprising ammonium salts and free ammonia, which is sent to the third-cycle section **32** and an aqueous stream comprising ammonium salts and free ammonia, in particular about 44 weight% of nitrogen, expressed as ammonia, 26 weight% of carbon, expressed as carbon dioxide, and less than 1 weight% of urea, which is produced by a condenser and is re-injected in the first-cycle section **30** via the return loop **24.** The third-cycle section **32** generates a concentrated urea solution depleted of ammonium salts, which is sent to the evaporation section **3,** and an aqueous composition comprising ammonium salts and free ammonia, in particular about 40 weight% of nitrogen, expressed as ammonia, 17 weight% of carbon, expressed as carbon dioxide, and less than 1 weight% of urea which is produced by a condenser, and is re-injected in the second-cycle section **31** via the return loop **25.** The evaporation section **3** produces a urea melt with a low water content, for example from 1 to 5 weight%, which is sent to the finishing section **4** to produce urea-based solids, and a gaseous stream comprising mainly water, ammonia, carbon dioxide and urea carry over, which is condensed and the process condensate is sent to the hydrolyzer section **8.** The hydrolyzer section **8** treats the condensate to obtain clean process condensate and an aqueous stream comprising ammonium salts and free ammonia, in particular about 33 weight% of nitrogen, expressed as ammonia, 9 weight% of carbon, expressed as carbon dioxide, and less than 1 weight% of urea, that may be re-injected in the third-cycle section **32.** An aqueous solution for SNCR/SCR can be obtained in four locations **15, 16, 17** and **18,** and diluted with an aqueous solution **40** to provide a SNCR/SCR solution.

## Claims

1.  A method for producing a selective non-catalytic reduction/selective catalytic reduction (SNCR/SCR) solution, comprising the steps of:

    a) reacting ammonia and carbon dioxide in a reactor, thereby obtaining an aqueous composition comprising urea and ammonium salts;
    b) processing the aqueous composition obtained in step a), thereby obtaining a first aqueous solution comprising urea, and a second aqueous solution comprising ammonium salts; and
    c) diluting the second aqueous solution comprising ammonium salts obtained in step b) with a third aqueous solution, thereby producing the SNCR/SCR solution.

2.  The method according to claim 1, wherein the aqueous composition obtained in step c) comprises from 10 to 40 weight% of nitrogen, expressed as ammonia.

3.  The method according to claim 1 or 2, wherein the aqueous composition obtained in step c) comprises from 5.0 to 35 weight% of carbon, expressed as carbon dioxide.

4. The method according to any one of claims 1 to 3, wherein the aqueous composition obtained in step c) does not form crystals above 15°C.

5. The method according to any one of claims 1 to 4, wherein the aqueous composition obtained in step c) has a boiling point of at least 35 °C.

6. The method according to any one of claims 1 to 5, wherein step b) comprises generating the first aqueous solution comprising urea and a gaseous stream comprising water, ammonia, and carbon dioxide, from the aqueous composition comprising urea and ammonium salts obtained in step a), and condensing the gaseous stream comprising water, ammonia, and carbon dioxide into the second aqueous solution comprising ammonium salts.

7. The method according to claim 6, wherein generating the first aqueous solution comprising urea and a gaseous stream comprising water, ammonia, and carbon dioxide, from the aqueous composition comprising urea and ammonium salts obtained in step a), is achieved by heating up the aqueous composition comprising urea and ammonium salts.

8. The method according to any one of claims 1 to 7, wherein the amount of nitrogen comprised in the SNCR/SCR solution represents from 0.5 to 10 % of the amount of nitrogen, introduced as ammonia, in a urea-producing plant.

9. The method according to any one of claims 1 to 8, wherein the aqueous composition obtained in step c) comprises from 0.01 to 5.0 weight% of urea.

10. The method according to any one of claims 1 to 8, wherein the third aqueous solution used in step c) is selected from the group consisting of demineralized water, steam condensate, process condensate, and any mixture thereof.

11. Use of an aqueous stream comprising ammonium salts from a urea-producing plant for producing a SNCR/SCR solution.

12. A urea-producing plant comprising:

- a high pressure section (1);
- a low pressure section (2); and
- a return line (20) for transporting a first aqueous solution comprising ammonium salts from the low pressure section (2) to the high pressure section (1);

**characterized in that** the urea-producing plant further comprises:

- a means for drawing out an amount of the first aqueous solution from the return line (20); and
- a means for mixing the first aqueous solution with a second aqueous solution.

13. A urea-producing plant comprising:

- a high pressure section (1);
- a low pressure section (2);
- a finishing section (4); and
- a return line for transporting a first aqueous solution comprising ammonium salts from the finishing section (4) to the low pressure section (2);

**characterized in that** the urea-producing plant further comprises:

- a means for drawing out an amount of the first aqueous solution from the return line; and
- a means for mixing the first aqueous solution with a second aqueous solution.

14. The urea-producing plant according to claim 12 or 13, further comprising:

- a medium pressure section (7);
- a return line (22) for transporting a third aqueous solution comprising ammonium salts from the low pressure section (2) to the medium pressure section (7);

- a means for drawing out an amount of the third aqueous solution from the return line (22); and
- a means for mixing the third aqueous solution with a fourth aqueous solution.

15. Method for adapting an existing urea-producing plant, comprising the steps of:

- installing a means for drawing out an amount of a first aqueous solution comprising ammonium salts;
- installing a means for mixing the first aqueous solution with a second aqueous solution.

**Patentansprüche**

1. Verfahren zum Herstellen einer SNCR-/SCR-Lösung (selektive nichtkatalytische Reduktion/selektive katalytische Reduktion), umfassend die folgenden Schritte:

a) Umsetzen von Ammoniak und Kohlenstoffdioxid in einem Reaktor, wodurch eine wässrige Zusammensetzung, die Harnstoff und Ammoniumsalze umfasst, erhalten wird;
b) Verarbeiten der in Schritt a) erhaltenen wässrigen Zusammensetzung, wodurch eine erste wässrige Lösung, die Harnstoff umfasst, und eine zweite wässrige Lösung, die Ammoniumsalze umfasst, erhalten werden; und
c) Verdünnen der in Schritt b) erhaltenen zweiten wässrigen Lösung, die Ammoniumsalze umfasst, mit einer dritten wässrigen Lösung, wodurch die SNCR/SCR-Lösung erhalten wird.

2. Verfahren nach Anspruch 1, wobei die in Schritt c) erhaltene wässrige Zusammensetzung zu 10 bis 40 Gew.-% Stickstoff, dargestellt als Ammoniak, umfasst.

3. Verfahren nach Anspruch 1 oder 2, wobei die in Schritt c) erhaltene wässrige Zusammensetzung zu 5,0 bis 35 Gew.-% Kohlenstoff, dargestellt als Kohlenstoffdioxid, umfasst.

4. Verfahren nach einem der Ansprüche 1 bis 3, wobei die in Schritt c) erhaltene wässrige Zusammensetzung über 15 °C keine Kristalle bildet.

5. Verfahren nach einem der Ansprüche 1 bis 4, wobei die in Schritt c) erhaltene wässrige Zusammensetzung einen Siedepunkt von mindestens 35 °C aufweist.

6. Verfahren nach einem der Ansprüche 1 bis 5, wobei Schritt b) das Erzeugen der ersten wässrigen Lösung, die Harnstoff umfasst, und eines gasförmigen Stroms, der Wasser, Ammoniak und Kohlenstoffdioxid umfasst, aus der in Schritt a) erhaltenen wässrigen Zusammensetzung, die Harnstoff und Ammoniumsalze umfasst, und das Kondensieren des gasförmigen Stroms, der Wasser, Ammoniak und Kohlenstoffdioxid umfasst, zu der zweiten wässrigen Lösung, die Ammoniumsalze umfasst, umfasst.

7. Verfahren nach Anspruch 6, wobei das Erzeugen der ersten wässrigen Lösung, die Harnstoff und einen gasförmigen Strom, der Wasser, Ammoniak und Kohlenstoffdioxid umfasst, aus der in Schritt a) erhaltenen wässrigen Zusammensetzung, die Harnstoff und Ammoniumsalze umfasst, durch Erwärmen der wässrigen Zusammensetzung, die Harnstoff und Ammoniumsalze umfasst, erzielt wird.

8. Verfahren nach einem der Ansprüche 1 bis 7, wobei die Menge an Stickstoff in der SNCR/SCR-Lösung 0,5 bis 10 % der Menge an Stickstoff, eingebracht als Ammoniak, in einer Harnstoff herstellenden Anlage ausmacht.

9. Verfahren nach einem der Ansprüche 1 bis 8, wobei die in Schritt c) erhaltene wässrige Zusammensetzung zu 0,01 bis 5,0 Gew.-% Harnstoff umfasst.

10. Verfahren nach einem der Ansprüche 1 bis 8, wobei die in Schritt c) verwendete dritte wässrige Lösung aus der Gruppe bestehend aus demineralisiertem Wasser, Dampfkondensat, Prozesskondensat und beliebigen Gemischen davon ausgewählt ist.

11. Verwendung eines wässrigen Stroms, der Ammoniumsalze umfasst, aus einer Harnstoff herstellenden Anlage zum Herstellen einer SNCR-/SCR-Lösung.

12. Harnstoff herstellende Anlage, umfassend:

- einen Abschnitt mit hohem Druck (1);
- einen Abschnitt mit niedrigem Druck (2); und
- eine Rücklaufleitung (20) zum Transportieren einer ersten wässrigen Lösung, die Ammoniumsalze umfasst, von dem Abschnitt mit niedrigem Druck (2) zu dem Abschnitt mit hohem Druck (1);

**dadurch gekennzeichnet, dass** die Harnstoff herstellende Anlage ferner Folgendes umfasst:

- ein Mittel zum Herausziehen einer Menge der ersten wässrigen Lösung aus der Rücklaufleitung (20); und
- ein Mittel zum Mischen der ersten wässrigen Lösung mit einer zweiten wässrigen Lösung.

13. Harnstoff herstellende Anlage, umfassend:

- einen Abschnitt mit hohem Druck (1);
- einen Abschnitt mit niedrigem Druck (2);
- einen Endbearbeitungsabschnitt (4); und
- eine Rücklaufleitung zum Transportieren einer ersten wässrigen Lösung, die Ammoniumsalze umfasst, von dem Endbearbeitungsabschnitt (4) zu dem Abschnitt mit niedrigem Druck (2);

**dadurch gekennzeichnet, dass** die Harnstoff herstellende Anlage ferner Folgendes umfasst:

- ein Mittel zum Herausziehen einer Menge der ersten wässrigen Lösung aus der Rücklaufleitung; und
- ein Mittel zum Mischen der ersten wässrigen Lösung mit einer zweiten wässrigen Lösung.

14. Harnstoff herstellende Anlage nach Anspruch 12 oder 13, ferner umfassend:

- einen Abschnitt mit mittlerem Druck (7);
- eine Rücklaufleitung (22) zum Transportieren einer dritten wässrigen Lösung, die Ammoniumsalze umfasst, von dem Abschnitt mit niedrigem Druck (2) zu dem Abschnitt mit mittlerem Druck (7);
- ein Mittel zum Herausziehen einer Menge der dritten wässrigen Lösung aus der Rücklaufleitung (22); und
- ein Mittel zum Mischen der dritten wässrigen Lösung mit einer vierten wässrigen Lösung.

15. Verfahren zum Anpassen einer existierenden Harnstoff herstellenden Anlage, umfassend die folgenden Schritte:

- Installieren eines Mittels zum Herausziehen einer Menge einer ersten wässrigen Lösung, die Ammoniumsalze umfasst;
- Installieren eines Mittels zum Mischen der ersten wässrigen Lösung mit einer zweiten wässrigen Lösung.

**Revendications**

1. Procédé de production d'une solution de réduction non catalytique sélective/réduction catalytique sélective (SNCR/SCR), comprenant les étapes de :

a) mise en réaction d'ammoniac et de dioxyde de carbone dans un réacteur, obtenant ainsi une composition aqueuse comprenant de l'urée et des sels d'ammonium ;
b) traitement de la composition aqueuse obtenue à l'étape a), obtenant ainsi une première solution aqueuse comprenant de l'urée, et une deuxième solution aqueuse comprenant des sels d'ammonium ; et
c) dilution de la deuxième solution aqueuse comprenant des sels d'ammonium obtenus à l'étape b) avec une troisième solution aqueuse, produisant ainsi la solution de SNCR/SCR.

2. Procédé selon la revendication 1, dans lequel la composition aqueuse obtenue dans l'étape c) comprend de 10 à 40 % en poids d'azote, exprimé comme ammoniac.

3. Procédé selon la revendication 1 ou 2, dans lequel la composition aqueuse obtenue dans l'étape c) comprend de 5,0 à 35 % en poids de carbone, exprimé comme dioxyde de carbone.

4. Procédé selon l'une quelconque des revendications 1 à 3, dans lequel la composition aqueuse obtenue à l'étape c) ne forme pas de cristaux au-dessus de 15 °C.

5. Procédé selon l'une quelconque des revendications 1 à 4, dans lequel la composition aqueuse obtenue dans l'étape c) a un point d'ébullition d'au moins 35 °C.

6. Procédé selon l'une quelconque des revendications 1 à 5, dans lequel l'étape b) comprend la génération de la première solution aqueuse comprenant de l'urée et un flux gazeux comprenant de l'eau, de l'ammoniac et du dioxyde de carbone, à partir de la composition aqueuse comprenant de l'urée et des sels d'ammonium obtenue dans l'étape a), et la condensation du flux gazeux comprenant de l'eau, de l'ammoniac et du dioxyde de carbone dans la seconde solution aqueuse comprenant des sels d'ammonium.

7. Procédé selon la revendication 6, dans lequel la génération de la première solution aqueuse comprenant de l'urée et un flux gazeux comprenant de l'eau, de l'ammoniac et du dioxyde de carbone, à partir de la composition aqueuse comprenant de l'urée et des sels d'ammonium obtenue dans l'étape a), est réalisée en chauffant la composition aqueuse comprenant de l'urée et des sels d'ammonium.

8. Procédé selon l'une quelconque des revendications 1 à 7, dans lequel la quantité d'azote comprise dans la solution SNCR/SCR représente de 0,5 à 10 % de la quantité d'azote, introduit comme ammoniac, dans une installation de production d'urée.

9. Procédé selon l'une quelconque des revendications 1 à 8, dans lequel la composition aqueuse obtenue dans l'étape c) comprend de 0,01 à 5,0 % en poids d'urée.

10. Procédé selon l'une quelconque des revendications 1 à 8, dans lequel la troisième solution aqueuse utilisée dans l'étape c) est choisie dans le groupe constitué par de l'eau déminéralisée, un condensat de vapeur d'eau, un condensat de procédé et n'importe quel mélange de ceux-ci.

11. Utilisation d'un flux aqueux comprenant des sels d'ammonium issue d'une installation de production d'urée pour produire une solution SNCR/SCR.

12. Installation de production d'urée comprenant :

   - une section haute pression (1) ;
   - une section basse pression (2) ; et
   - une ligne de retour (20) pour transporter une première solution aqueuse comprenant des sels d'ammonium depuis la section basse pression (2) vers la section haute pression (1) ;

   **caractérisée en ce que** l'installation de production d'urée comprend en outre :

   - un moyen de prélèvement d'une quantité de la première solution aqueuse dans la ligne de retour (20) ; et
   - un moyen de mélange de la première solution aqueuse avec une seconde solution aqueuse.

13. Installation de production d'urée comprenant :

   - une section haute pression (1) ;
   - une section basse pression (2) ;
   - une section de finition (4) ; et
   - une ligne de retour pour le transport d'une première solution aqueuse comprenant des sels d'ammonium depuis la section de finition (4) vers la section basse pression (2) ;

   **caractérisée en ce que** l'installation de production d'urée comprend en outre :

   - un moyen de prélèvement d'une quantité de la première solution aqueuse dans la ligne de retour ; et
   - un moyen de mélange de la première solution aqueuse avec une seconde solution aqueuse.

14. Installation de production d'urée selon la revendication 12 ou 13, comprenant en outre :

   - une section moyenne pression (7) ;
   - une ligne de retour (22) pour transporter une troisième solution aqueuse comprenant des sels d'ammonium depuis la section basse pression (2) vers la section moyenne pression (7) ;

- un moyen de prélèvement d'une quantité de la troisième solution aqueuse de la ligne de retour (22) ; et
- un moyen de mélange de la troisième solution aqueuse avec une quatrième solution aqueuse.

15. Procédé d'adaptation d'une installation existante de production d'urée comprenant les étapes de :

- installation d'un moyen de prélèvement d'une quantité d'une première solution aqueuse comprenant des sels d'ammonium ;
- installation d'un moyen de mélange de la première solution aqueuse avec une seconde solution aqueuse.

*Figure 1*

*Figure 2*

Figure 3